Europäisches Patentamt

European Patent Office

Office européen des brevets

(19)

(11) Veröffentlichungsnummer : **0 437 274 B1**

(12)  # EUROPÄISCHE PATENTSCHRIFT

(45) Veröffentlichungstag der Patentschrift :
**18.05.94 Patentblatt 94/20**

(51) Int. Cl.$^5$ : **A61K 33/10**

(21) Anmeldenummer : **91100318.4**

(22) Anmeldetag : **11.01.91**

---

(54) **Bicarbonat und Calcium enthaltende Infusions- und Dialysierlösung.**

---

(30) Priorität : **12.01.90 DE 4000789**

(43) Veröffentlichungstag der Anmeldung :
**17.07.91 Patentblatt 91/29**

(45) Bekanntmachung des Hinweises auf die
Patenterteilung :
**18.05.94 Patentblatt 94/20**

(84) Benannte Vertragsstaaten :
**AT BE CH DE DK ES FR GB GR IT LI NL SE**

(56) Entgegenhaltungen :
**EP-A- 0 086 553**
**EP-A- 0 347 714**
**WO-A-86/00239**
**ASAIO TRANSACTIONS. vol. 32, no. 1, 1986,**
**HAGERSTOWN, MD US Seiten 422 - 424;M.**
**Feriani et al.: "Buffer Balance in Bicarbonate**
**Hemofiltration"**

(73) Patentinhaber : **NEPHRO-MEDICA**
**PHARMAZEUTISCHE**
**VERTRIEBSGESELLSCHAFT MBH**
**Giessener Strasse 115**
**D-35440 Linden (DE)**

(72) Erfinder : **Zimmermann, Eckhard, Dr.**
**Wilhelm Leuschner Strasse 14**
**W-6800 Mannheim 1 (DE)**

(74) Vertreter : **Schubert, Siegmar, Dipl.-Ing. et al**
**Patentanwälte Dr. V. Schmied-Kowarzik Dr. P.**
**Weinhold Dipl.-Ing. G. Dannenberg Dr. D.**
**Gudel Dipl.-Ing. S. Schubert Dr. P. Barz Grosse**
**Eschenheimer Strasse 39**
**D-60313 Frankfurt (DE)**

EP 0 437 274 B1

## Beschreibung

Die Erfindung betrifft eine Infusions- und Dialysierlösung, enthaltend Bicarbonat und Calciumionen und gegebenenfalls weitere Elektrolyte und Zusatzstoffe nach dem Oberbegriff des Anspruchs 1 bzw. 2.

Ein weiterer Aspekt der Erfindung betrifft ein Verfahren zur Herstellung einer solchen Infusions- und Dialysierlösung.

Patienten mit chronischer Niereninsuffizienz und Dialysepatienten weisen im allgemeinen eine metabolische Acidose (urämische Acidose) auf. Verantwortlich dafür ist die bei eingeschränkter Nierenfunktion verminderte Ausscheidung von Wasserstoffionen ($H^+$). Diese Acidose ist zunächst charakterisiert durch einen erniedrigten Blut-pH-Wert (unter 7,38), ein vermindertes Standard-Bicarbonat sowie ein Basendefizit. Metabolische Konsequenzen der Acidose sind eine Stimulation des Proteinabbaus, insbesondere durch eine Stimulation des Abbaus verzweigtkettiger Aminosäuren wie z.B. Leucin und eine Stimulation der Parathormon (PTH) Sekretion verbunden mit einer Knochenentkalkung und Induktion einer Hyperphosphatämie.

Metabolische (nicht-respiratorische) Acidosen können, außer bei Niereninsuffizienz, als Ursache einer Störung des Säuren-Basen-Haushalts auch als Ketoacidosen bei Diabetes Mellitus, Hunger, Verzweigtkettenkrankheit und Isovalerianämie, als Laktatacidose bei Sauerstoffmangel und Glykogenspeicherkrankheiten vom Typ I, als Formiatacidose nach Methanolvergiftung oder als Subtraktionsacidosen bei Diarrhoen und Erbrechen auftreten.

Eine absolute Therapieindikation stellt die metabolische Acidose dann dar, wenn sie dekompensiert und sich durch Hyperventilation, Übelkeit, Somnolenz oder Anstieg des Serumkaliums manifestiert. Das Standard-Bicarbonat im Serum liegt dann in der Regel unter 15 mmol/l.

Die Korrektur der Acidose wird bisher durch den Übertritt von $H^+$-Ionen aus dem Blut ins Dialysat bei Dialysepatienten als auch durch Diffusion von Puffersubstanzen oder Substanzen, deren Metabolite Puffersubstanzen bilden, aus der Spüllösung (bei Dialysepatienten), oder allgemein Infusionslösung, ins Blut zu erreichen versucht. Weiterhin besteht die Möglichkeit bei Dialysepatienten und Nicht-Dialysepatienten den Säure-Basen-Haushalt über die orale Verabreichung $H^+$-Ionen-neutralisierender Substanzen zu korrigieren. An intravenös zu verabreichenden Infusionslösungen zur Acidosekorrektur hat sich in der Praxis eine Natriumhydrogencarbonatlösung, jedoch ohne Calciumionen, bewährt.

Nachdem zunächst Natriumbicarbonat in Dialysier- und Spüllösungen als Puffer verwendet worden war, wurde Mitte der 60er Jahre Acetat als Dialysatpuffer eingeführt, da dieser zusammen mit Calcium, im Gegensatz zu Bicarbonat, in der Lösung stabil war, wenn der pH-Wert der Lösung nicht mit z.B. Oxycarbon gesenkt wurde. Acetat wirkt als Puffer, indem es vorwiegend in der Leber zu Bicarbonat (physiologischer Puffer im Blut) metabolisiert wird. Einem gleichartigen Abbau unterliegt auch Laktat, welches in Spüllösungen zur kontinuierlichen ambulanten Peritonealdialyse (CAPD) eingesetzt wird. Allerdings ist der gewünschte Abbau von Acetat oder Laktat nicht bei allen Patienten in befriedigendem Maße garantiert bzw. die zur ausreichenden Bicarbonatbildung notwendigen Konzentrationen praktisch nicht zu realisieren. Eine Acetatüberlastung führt zu Kreislaufproblemen.

Die damals wie heute nicht beherrschbare Stabilität Bicarbonat enthaltender Dialysierlösungen war, insbesondere durch die Problematik der Ausfällung von Calciumcarbonat in diesen Lösungen, der Grund für den Austausch der Puffersubstanzen.

Ein weiteres, neueres Verfahren ist die Bicarbonat-Dialyse, bei der wiederum Bicarbonat als Puffer verwendet wird. Jedoch werden hierzu zwei Dialysekonzentrate, von denen das eine (basische Lösung) Bicarbonat und NaCl, das andere (saure Lösung) Calcium und weitere Elektrolyte und Essigsäure bzw. Acetat enthält, verwendet. Diese beiden Konzentrate werden unmittelbar vor der Anwendung kontinuierlich gemischt, da ansonsten wiederum Calciumcarbonat ausfällt. Das Ausfallen geringer Mengen Calciumcarbonat läßt sich allerdings auch bei diesem Verfahren nicht vermeiden, wodurch es in der Praxis der Anwendung regelmäßig zu unerwünschten Kalkablagerungen in den Dialysemaschinen kommt. Ein weiterer Nachteil von Bicarbonat-Konzentraten ist eine in der wissenschaftlichen Literatur ausführlich dokumentierte Pyrogenbelastung solcher Konzentrate, die bei Dialysepatienten zu anaphylaktoiden Reaktionen führen kann.

Als orale $H^+$-neutralisierende Substanzen werden Salze des Bicarbonats, wie z.B. Calciumcarbonat, Natriumhydrogencarbonat (Natron) oder Natriumbicarbonat eingesetzt, ferner Magnesium- bzw. Aluminiumhydroxid. Der Nachteil dieser Substanzen bei Verabreichung als Acidosetherapeutika ist die damit verbundene hohe Belastung des Organismus mit den entsprechenden Metallanteilen dieser Verbindungen (Calcium, Natrium, Magnesium oder Aluminium). Daraus resultierende bekannte Krankheitsbilder sind z.B. Hypercalcämien und Nephrocalcinose, Hypernatriämien und Bluthochdruck, Hypermagnesiämien und Aluminiumbelastung, die zu dem bekannten Erkrankungsbild der urämischen Encephalopathie führen können. Leichtere Acidosefälle werden oral auch mit Citrat- oder Gluconatsalzen, überwiegend als Calciumsalze, behandelt. Der Einsatz der letzteren Substanzen ist jedoch bei mittleren und schweren Acidosen und bei chronischer Acidosebehandlung (chronische Niereninsuffizienz) nicht ausreichend.

Infusionslösungen zur Acidosetherapie enthalten Carbonate, wie z.B. Natriumhydrogencarbonat oder andere im basischen Bereich wirksame Puffer, wie z.B. Trispuffer.

Gemäß den medizinischen Anforderungen an Infusions- und Dialysierlösungen zur chronischen Acidosebehandlung, insbesondere bei Dialysepatienten, muss eine solche Lösung, um langfristig einen wirksamen antiacidotischen Effekt zu erzielen und keine weiteren Störungen im Elektrolythaushalt hervorzurufen, üblicherweise Calciumionenkonzentrationen von etwa 1,75 mmol/l und Bicarbonationen von wenigstens 30 mmol/l, sowie gegebenenfalls weitere Elektrolyte in blutphysiologischen Mengen und weitere Zusätze, enthalten. Durch solche notwendig hohen Calcium- und Bicarbonatkonzentrationen kommt es, begünstigt durch die Art und Verwendung bzw. des Einsatzes dieser Lösungen, zu einem Überschreiten des Löslichkeitsproduktes von Calcium und Carbonat und somit zur Präzipitation (Ausfällen) von Calciumcarbonat in der Lösung, mit der Folge technischer (Verkalkung von Dialysemaschinen) und medizinischer (Erniedrigung der freien und somit verfügbaren Calcium- und Bicarbonationenkonzentration) Probleme.

Die Präzipitation von Calciumcarbonat in Calciumionen und Bicarbonationen enthaltenden wässrigen Lösungen läßt sich chemisch folgendermaßen erklären.

Calcium, aber auch andere divalente Kationen wie z.B. Magnesium, sind sogenannte Härtebildner, d.h. sie bilden mit anderen divalenten Anionen, wie z.B. Carbonat, unlösliche Salze. So reagieren abhängig von der jeweiligen Ionenkonzentration Calciumionen mit Carbonationen nach der Reaktionsgleichung

$$Ca^{++} + CO_3^{--} \longrightarrow CaCO_3 \qquad \text{(Gleichung 1)}$$

zu unlöslichem Calciumcarbonat. Dieser Reaktionsschritt ist abhängig vom Löslichkeitsprodukt LP, dem pH-Wert der Lösung, der Temperatur und dem Druck.

Das Löslichkeitsprodukt $LP_{CaCO3}$ für Calciumcarbonat ist definiert als das Produkt aus der Calcium- und Carbonationenkonzentration und findet sich in einschlägigen chemischen Tabellenwerken, wie z.B. 'Handbook of Chemistry and Physics. Hrsg.: Weast, R.C, CRC Press, Cleveland, 1974',

$$LP_{CaCO3} = [Ca^{++}] \times [CO_3^{--}] = 0,99 \times 10^{-8} \text{ (bei 15° C)} \qquad \text{(Gleichung 2)}.$$

Dies bedeutet, daß bei einer weiteren Erhöhung einer der Konzentrationen unlösliches Calciumcarbonat ausfällt (gesättigte Lösung).

Für Magnesium ist $LP_{MgCO3} = 2,6 \times 10^{-5}$ (bei 12° C) und damit wesentlich größer als $LP_{CaCO3}$, so daß Carbonatpräzipitationen in Calium- und Magnesiumionen enthaltenden Infusions- und Dialysierlösung nur durch Calcium verursacht werden.

Der Einfluß des pH-Wertes auf Gleichung (1) ist aus der Henderson-Hasselbalch-Gleichung ersichtlich:

$$pH = pK + \log \frac{[\text{konjugierte Base}]}{[\text{Säure}]} \qquad \text{(Gleichung 3)}.$$

Im Falle eines Kohlendioxid-/Bicarbonat-Puffersystems stellt sich ein Gleichgewicht nach folgender Reaktionsgleichung ein:

$$CO_2 + H_2O \Longleftrightarrow (H_2CO_3) \Longleftrightarrow HCO_3^- + H^+ \Longleftrightarrow CO_3^{--} + 2H^+ \qquad \text{(Gleichung 4)}.$$

Im basischen Milieu ist Gleichung (4) nach rechts verschoben und es bildet sich aus 2 $OH^-$ (Base) + 2 $H^+$ (aus Reaktionsgleichung) 2 $H_2O$; durch einen verbleibend hohen Carbonatanteil ($CO_3^{--}$) ist die Gefahr einer Calciumcarbonatpräzipitation erhöht (vgl. Gleichung 1).

Im sauren Milieu ist Gleichung 4 nach links verschoben und Kohlendioxid ($CO_2$) entweicht; die $CO_3^{--}$-Konzentration ist erniedrigt.

Im ersten Dissoziationsschritt ist hinsichtlich der Henderson-Hasselbalch-Gleichung $HCO_3^-$ (Bicarbonat = Hydrogencarbonat) die konjugierte Base und $CO_2$ (Kohlendioxid) die Säure, wobei Kohlendioxid für $CO_2$ und $H_2CO_3$ (Kohlensäure) steht, da z.B. bei 37° C nur 1/400 des gesamten Kohlendioxids in hydratisierter Form als $H_2CO_3$ vorliegt. Deshalb gilt

$$[CO_2 + H_2CO_3] \approx [CO_2] \qquad \text{(Gleichung 5)}.$$

Im zweiten Dissoziationsschritt ist $CO_3^{--}$ (Carbonat) die konjugierte Base und $HCO_3^-$ (Hydrogencarbonat) die Säure.

Aus Gleichung (3) ergibt sich somit für ein Kohlendioxid-/Bicarbonat-Puffersystems in der ersten Stufe:

$$pH = pK_1 + \log \frac{[HCO_3^-]}{[CO_2]}, \text{ wobei } pK_1 \approx 6,4 \text{ (bei 25° C)} \qquad \text{(Gleichung 6)}$$

und in der zweiten Stufe:

$$pH = pK_2 + \log \frac{[CO_3^{--}]}{[HCO_3^-]}, \text{ wobei } pK_2 \approx 10,3 \text{ (bei 25°C)} \qquad \text{(Gleichung 7)}.$$

Aus Gleichung (6) und (7) wird deutlich, daß die Carbonatkonzentration durch die Bikarbonatkonzentration und den pH-Wert und umgekehrt der pH-Wert von der Carbonat bzw. Bikarbonatkonzentration bestimmt werden. Gleichzeitig wird deutlich, daß bei genügend niedrigem pH-Wert,

3

also $H^+$-Überschuß, eine Präzipitation von Calciumcarbonat in der Weise verhindert wird, daß analog Gleichung (1) nicht Calciumcarbonat, sondern das leicht lösliche Calciumhydrogencarbonat nach folgender Reaktionsgleichung gebildet wird:

$$Ca^{++} + 2CO_3^{--} + 2H^+ ----> Ca(HCO_3)_2 \qquad \text{(Gleichung 8).}$$

Die Temperatur beeinflußt die Höhe des pK-Wertes umgekehrt proportional, d.h. bei steigender Temperatur sinkt der pK-Wert und somit der pH-Wert. So findet sich in den wissenschaftlichen Tabellen z.B. für den in Gleichung (6) wiedergegebenen $pK_1$-Wert ein Wert von 6,4 bei 25° C und von 6,1 bei 37° C.

Eine besondere Bedeutung kommt der Aktivität des gelösten $CO_2$ zu, da gemäß der Definition der pK-Werte zu deren Bestimmung die Aktivitätskoeffizienten (= Löslichkeitskoeffizient $\alpha$) herangezogen werden. Kohlensäure zerfällt in Wasser und $CO_2$, das, in Wasser gelöst, einen Kohlendioxidpartialdruck $pCO_2$ verursacht. Der Löslichkeitskoeffizient für $CO_2$ in Wasser beträgt $\alpha_{pCO2}$ = 0,0306 mmol/l/mm Hg (1 Pa = 7,3 x $10^{-3}$ mm Hg) oder

$$[CO_2]_{aktiv} = 0,0306 \times pCO_2 \qquad \text{(Gleichung 9).}$$

Gleichung (6) bekommt so die Form

$$pH = pK_1 + \log \frac{[HCO_3]^{aktiv}}{0,0306 \times pCO_2}, \text{wobei } pK_1 \approx 6,4 \ (25° C) \qquad \text{(Gleichung 10).}$$

Aus Gleichung (8) wird deutlich, daß durch eine Erniedrigung des $pCO_2$ der pH-Wert in der Lösung ansteigt. Dadurch wird das in Gleichung (4) dargestellte Gleichgewicht zugunsten einer Konzentrationserhöhung von $CO_3^{--}$ verschoben, wodurch das Löslichkeitsprodukt $LP_{CaCO3}$ für Calciumcarbonat überschritten wird, mit der Folge einer Präzipitation von Calciumcarbonat.

Aus dem Stand der Technik sind in den Verfahren der Blutreinigung (Hämodialyse mit verwandten Verfahren und Peritonealdialyse) schon seit mehreren Jahren Bicarbonat als Puffer enthaltende Dialysierflüssigkeiten in der Weise hergestellt worden, daß einerseits ein basisches Bicarbonatkonzentrat und andererseits ein saures Calciumionen enthaltendes Elektrolytkonzentrat in getrennten Behältnissen gehalten wird. Diese beiden Lösungen werden unmittelbar vor dem Gebrauch gemischt. Dennoch erfolgen selbst bei dieser Art der Herstellung und des sofortigen Einsatzes entweder als Dialysierflüssigkeit in Hämodialysemaschinen oder als Dialysierflüssigkeit zur Peritonealdialyse, Calciumcarbonatpräzipitationen, wenn die Calciumkonzentration notwendigerweise bei ca. 1,75 mmol/l und die Bicarbonatkonzentration über 30 mmol/l liegt. Technisch hat dies Störungen im Dialysebetrieb durch Verkalken von Dialysemaschinen und medizinisch eine inadequate Azidosekorrektur und ungenügende Zufuhr von Calciumionen zur Folge.

Desweiteren sind eine Reihe von wissenschaftlichen Publikationen und von Patentschriften bekannt, die sich mit der Herstellung von Calcium und Bicarbonat enthaltenden Infusions- und Dialysierlösungen beschäftigen. Gemeinsam ist allen Druckschriften, daß jeweils der Einsatz zweier getrennter Lösungen, eines basischen Bicarbonatkonzentrats und einer sauren, Calciumionen enthaltenden Elektrolytlösung, vorgeschlagen wird. Durch Mischen entsprechender Anteile dieser zwei Lösungen wird anschließend der pH-Wert im physiologischen Bereich von ca. 7,4 eingestellt. Diesbezügliche Patentschriften zur Herstellung solcher Lösungen sind z.B. in der EP-OS 086 553, der EP-OS 161 471, der DE-OS 31 46 425 und der EP-OS 022 922 beschrieben. Allen Publikationen ist gemein, daß sie keinerlei Beeinflussung des $pCO_2$ oder des pH-Wertes der Bicarbonatlösung vorschlagen.

In medizinischen und naturwissenschaftlichen Gebieten werden routinemäßig seit Jahrzehnten stabile Calcium und Bicarbonat enthaltende Pufferlösungen, wie z.B. Krebs-Bicarbonat-Puffer, in der Weise hergestellt und verwendet, daß die Calciumcarbonatpräzipitation durch ständiges Begasen der Lösung mit $CO_2$ verhindert wird.

Wesentlich ist hierbei also die Aufrechterhaltung eines genügend hohen $pCO_2$. Ermöglicht wird dies durch die kontinuierliche Begasung in einem offenen System, ganz im Gegensatz zu geschlossenen Systemen, die aber bei der Anwendung von Infusions- und Dialysierlösungen obligat sind. Solche sogenannten geschlossenen Systeme, wie z.B. Plastikbehältnisse für Infusions- und Dialysierlösungen, zeichnen sich jedoch größtenteils dadurch aus, daß sie für Gase, also auch für $CO_2$, teilweise permeabel sind. Dadurch entweicht mehr oder weniger $CO_2$, z.B. bei der Lagerung der Infusions- und Dialysierlösung. Spätestens jedoch bei dem Gebrauch solcher Lösungen, z.B. nach dem Mischen o.g. Dialysierlösungen, wird das geschlossene System geöffnet und es entweicht $CO_2$. Der $pCO_2$ erniedrigt sich folglich, der pH-Wert steigt, wodurch die Gefahr für Calciumcarbonatpräzipitationen erhöht wird.

Aus Gleichung (10) ist ersichtlich, daß durch die Maßnahme der kontinuierlichen $CO_2$-Begasung der pH-Wert der Lösung erniedrigt wird und somit, wie bereits erwähnt, lösliches Calciumhydrogencarbonat entsteht. Es handelt sich also bekanntermaßen um ein Ansäuern der Lösung. Selbstverständlich kann dieses Ansäuern auch mit mineralischen Säuren, wie z.B. Salzsäure (HCl), oder mit organischen Säuren, wie z.B. Essigsäure und Milchsäure, erfolgen. Der Nachteil bei einer derartigen Vorgehensweise liegt darin, daß letztendlich nicht

4

der pH-Wert der Lösung, sondern der $pCO_2$ für die Stabilität der Lösung verantwortlich ist. Das bedeutet, daß bei dem unvermeidbaren Entweichen von $CO_2$, z.B. beim Öffnen des Systems, der pH-Wert durch ein Absinken des $pCO_2$ wieder ansteigt, also das Reaktionsgleichgewicht nach Gleichung (4) nach rechts verschoben ist, wodurch vermehrt Carbonat entsteht (Gefahr der Calciumcarbonatausfällung). Nach Erschöpfen der Protonendonatorkapazität der zugeführten Säuren, d.h. bei entsprechender $CO_2$-Entweichung, kommt es zwangsläufig zum Überschreiten von $LP_{CaCO3}$.

Bei der Verwendung von HCl zum Ansäuern Bicarbonat enthaltender Infusions- und Dialysierlösungen, besteht darüberhinaus die Gefahr, bei intrakorporealer Verabreichung solcher Infusions- und Dialysierlösungen, Hyperchloridämien bei den Patienten zu induzieren, da erfahrungsgemäß solche Infusions- und Dialysierlösungen $Cl^-$-Konzentrationen über 120 mmol/l aufweisen (Blutnormalwert = 97 - 108 mmol/l).

Insofern wird deutlich, daß die kausale Problematik der Herstellung einer stabilen Calciumionen und Bicarbonationen enthaltenden Infusions- und Dialysierlösung darin liegt, den $pCO_2$ in einem Bereich konstant zu halten, der sicherstellt, daß keine Calciumcarbonatpräzipitationen auftreten.

Es ist bereits eine Calciumionen und Bicarbonat enthaltende Dialysierlösung der eingangs genannten Gattung bekannt, die durch Mischen eines bicarbonathaltigen Konzentrats mit einer sauren calciumhaltigen Lösung hergestellt wird und bei der ein Säuregehalt in der sauren Lösung so eingestellt wird, daß der pH-Wert der gebrauchsfertigen Lösung auch bei $CO_2$-Verlust in einem Bereich zwischen 7,2 und 7,4 bleibt (EP-A-0 086 553). Dabei unterbleibt eine Ausfällung unlöslicher Salze aus der Dialysierlösung.

Bekannt ist weiterhin ein Zusammenhang zwischen pH-Wert einer bestimmten Dialysierlösung und deren $CO_2$-Partialdruck, wobei der pH-Wert durch mehrere Faktoren, z.B. Luftkontakt und Alterung beeinflußt wird (Druckschrift A.S.A.I.O. Transactions).

Aufgabe der vorliegenden Erfindung ist es, eine andere Calcium und Bicarbonat enthaltende Infusions- und Dialysierlösung zur Verfügung zu stellen, bei der nicht die Gefahr besteht, daß in der gebrauchsfertigen Infusions- und Dialysierlösung eine Calciumcarbonatpräzipitation während der Anwendung auftritt, bei der außerdem eine genügend hohe Bicarbonatkonzentration in der Infusions- und Dialysierlösung gewährleistet wird, um metabolische Acidosen langfristig effektiv therapieren zu können, und weiterhin sichergestellt wird, daß in der Infusions- und Dialysierlösung eine genügend hohe Calciumionenkonzentration von ca. 1,75 mmol/l vorliegt.

Die Lösung der Aufgabe erfolgt durch die Zusammensetzung der gebrauchsfertigen Infusions- und Dialysierlösung gemäß dem kennzeichnenden Teil des Anspruchs 1 bzw. 2 unter Einsatz eines physiologischen Puffergemisches aus einem organischen Puffersystem und gegebenenfalls einem anorganischen Bestandteil wie im einzelnen weiter unten angegeben. Der $pCO_2$ in dieser Infusions- und Dialsysierlösung wird konstant über $87.8 \times 10^2$ Pa (66 mm Hg) gehalten. Der Bicarbonationengehalt beträgt wenigstens 30 mmol/l, und die Calciumionenkonzentration liegt bei ca. 1,75 mmol/l oder darunter.

Durch die konstante Anwesenheit eines Nicht-Kohlendioxid-Bicarbonat-Puffersystems in Form eines organischen physiologischen Puffersystems wird bei einer Entweichung von $CO_2$, wie z.B. nach Öffnen des die Infusions- und Dialysierlösung enthaltenden Behältnisses oder in Form von Diffusion von $CO_2$ durch Plastikmaterialien des Behältnisses $CO_2$ aufgrund einer $H^+$-Freisetzung oder einer OH-Aufnahme des organischen Puffersystems kontinuierlich nachgeliefert (vgl. Gleichung 4).

Dadurch wird gleichzeitig erreicht, daß der pH-Wert dieser Lösung im definiert eingestellten Bereich von beispielsweise 7,40 konstant gehalten wird.

Dieses erfindungsgemäße organische Puffersystem steht in Wechselwirkung mit dem Kohlensäure-Bicarbonat-Puffersystem.

Kohlensäure-Bicarbonat-Puffersystem (a) :

$$H^+ + HCO_3 - <===> CO_2 + H_2O, \text{ d.h. } pCO_2 \text{ steigt im Sauren}$$
$$OH^- + CO_2 <===> HCO_3^-, \text{ d.h. } pCO_2 \text{ fällt im Basischen}$$

Organisches Puffersystem (b) :

$$H^+ + Ac^- <===> HAc, (Ac = Carbonsäure)$$
$$OH^- + HAc <===> Ac^- + H_2O$$

Durch Kombination beider Puffersysteme wird erreicht, daß bei einem Absinken des $pCO_2$, also einem Anstieg des pH bei (a), $H^+$-Ionen aus (b) freigesetzt werden und dadurch der pH-Wert bzw. $pCO_2$ konstant gehalten werden.

Vorzugsweise finden als organische Puffersysteme Gemische einer organischen Säure oder deren Natrium-, Kalium-, Magnesium- oder Calciumsalze, insbesondere Carbonsäuren mit einer Kettenlänge von 3 bis 8 Kohlenstoffatomen, die vornehmlich 1- bis 3-wertig sind, Anwendung und die einen pK-Wert in der ersten oder zweiten oder dritten Stufe im Bereich von 5,0 bis 7,6 aufweisen und einem korrespondierenden Säuren- bzw. Basenpaar dieser Carbonsäuren oder einer anorganischen Base, ausgewählt aus der Gruppe Hydroxid und Phosphat, insbesondere Natrium-, Kalium-, Magnesium- oder Calciumhydroxid oder gegebenenfalls einer anorganischen Säure. Die zur Anwendung kommenden Konzentrationen des Puffergemisches liegen im Bereich von 10 bis 200 mmol/l.

Zur Herstellung des Puffergemisches finden vornehmlich physiologische Carbonsäuren, die im Citratcyclus als Metabolite vorkommen, Anwendung. Insbesondere sind dies Citrat, Isocitrat, Oxalsuccinat, $\alpha$-Ketoglutarat, Succinat, Fumarat, Malat, Oxalacetat und Pyruvat. Desweiteren können aber auch Aminosäuren, vornehmlich essentielle Aminosäuren oder deren $\alpha$-Ketoanaloge eingesetzt werden.

Im Citratzyklus ( = Krebszyklus nach H. A. Krebs, Oxford, Nobelpreis 1954), in den der Kohlenhydrat-, Eiweiß- bzw. Aminosäuren- und Fettstoffwechsel in Form des Pyruvats oder der aktivierten Essigsäure einmünden, nehmen diese Carbonsäuren eine dominierende Schlüsselstellung ein, indem der intermediäre Stoffwechsel dieser Nährstoffe somit über diese Carbonsäuren als Zwischenprodukt abläuft. Durch das beim Abbau entstehende Bicarbonat wird die renale Acidose günstig beeinflußt.

Die sekundäre (metabolische ) Bicarbonatbildung aus Carbonsäuren des Citratcyclus bei der erfindungsgemäßen Verwendung dieser Carbonsäuren als organisches Puffersystem wirkt somit als eine Art 'Nachbrenner'.

Durch diese physiologische Stellung dieser Carbonsäuren des Citratcyclus im Intermediärstoffwechsel ist die pharmakologisch toxikologische Unbedenklichkeit gewährleistet.

Die vorzugsweise Einstellung des $pCO_2$ auf wenigstens 66 mm Hg in der Infusions- und Dialysierlösung bei der erfindungsgemäßen Herstellung Calcium und Bicarbonat enthaltender Infusions- und Dialysierlösungen durch die erfindungsgemäße Verwendung eines organischen Puffersystems hat den Vorteil, daß die Carbonatkonzentration ($CO_3^{--}$ ) in dieser Lösung extrem niedrig liegt. Bei einer Bikarbonatkonzentration ($HCO_3^-$) von 35 mmol/ l und einem $pCO_2$ von 87.8 x 10²Pa (66 mm Hg) stellt sich nach Gleichung (10) bei einem pK1-Wert von 6,1 bei 37°C ein pH-Wert von

$$pH = 6,1 + \log \frac{[35]_{aktiv}}{0,0306 \times 66} = 7,34 \quad \text{(Gleichung 11)}$$

ein. Aus Gleichung (7) errechnet sich dann eine Carbonatkonzentration von $[CO_3^{--}] = 0,002$ mmol/l.

Bei einer $Ca^{++}$-Konzentration von 1,75 mmol/l errechnet sich analog Gleichung (2) ein Konzentrationsprodukt (KP) von

$$KP = [Ca^{++}] \times [CO_3^{--}] = 0,00175 \times 0,000002 = 3,5 \times 10^{-9}$$
$$<< LP_{CaCO3} = 0,99 \times 10^{-8} \quad \text{(Gleichung 12)}.$$

Da KP kleiner ist als das Löslichkeitsprodukt $LP_{CaCo3}$ besteht keine Gefahr einer Calciumcarbonatpräzipitation.

Im Rahmen der Erfindung wurde weiterhin festgestellt, daß es beim Mischen bekannter basischer Bicarbonat- und saurer Calciumionen enthaltender Elektrolytlösungen zwar berücksichtigt wurde, den pH-Wert im physiologischen Bereich von 7,2 bis 7,6 einzustellen, es jedoch vernachlässigt wurde, zu berücksichtigen, daß sich der beim Mischen bildende $CO_2$-Partialdruck, der wie oben ausgeführt, notwendig ist, um eine Calciumcarbonatpräzipitation zu verhindern, gemäß Gleichung (1) erst relativ langsam als Folge der chemischen Reaktion aufbaut und darüberhinaus auch nicht stabil bleibt, da das System offen ist. Dadurch kommt es beim Mischen und anschließend bei der Anwendung (Dialysemaschine) zu mehr oder weniger kurzfristigen Überschreitungen von $LP_{CaCO3}$, ausreichend um Calciumcarbonatkristallkeime zu bilden, die je nach weiteren physikalischen Umständen zu Calciumcarbonatausfällungen führen.

Aufgrund des durch das mit dem organischen Puffersystem eingestellten und konstant gehaltenen $pCO_2$ kommt es jedoch bei der erfindungsgemäßen Infusions- und Dialysierlösung zu keiner Zeit zu Überschreitungen des Löslichkeitsproduktes für $CaCO_3$. Selbst wenn in der Infusions- und Dialysierlösung gelöstes $CO_2$ entweicht, z.B. beim Öffnen des 'Bicarbonat-Calcium-Systems' , wird dadurch nicht der $pCO_2$ erniedrigt, da aufgrund des anwesenden organischen Puffersystems solange ständig $H^+$-Ionen freigesetzt werden, bis die Konzentration an $CO_2$ aus Gleichung (1) nachgeliefert wurde, die entwichen ist.

Eine gebrauchsfertige Infusions- und Dialysierlösung wird nach Anspruch 6 so hergestellt, daß in eine ein organisches Puffersystem und gegebenenfalls eine anorganische Base oder Säure als physiologisches Puffergemisch enthaltende wässrige Lösung eine (A) die gewünschte Menge Bicarbonationen enthaltende Lösung (B) und anschließend die gewünschte Calciumionen enthaltende Lösung (C) gegeben wird. Gewünschtenfalls können Nicht-Calcium-Eletrolytzusätze in (A), (B) oder (C) enthalten sein. Bicarbonat- und Calciumionen können auch direkt in Form ihrer Salze, z.B. als Natriumhydrogencarbonat und als Calciumchlorid in (A) gelöst werden. Umgekehrt kann auch das ein organisches Puffersystem und gegebenenfalls eine anorganischen Base oder Säure enthaltende Puffergemisch als Lösung oder als festes Salz zu (B) gegeben werden. Von Bedeutung ist jeweils, daß bei Zugabe von Calciumionen bereits eine wässrige Lösung von (A) und (B) vorliegt.

(A), (B) und (C) können im Bedarfsfall getrennt produziert, in geschlossene Behältnisse abgefüllt und sterilisiert werden und erst vor Gebrauch gemischt werden, oder (A) und (B) werden vorgemischt oder (A) und (C) werden vorgemischt oder (A) und (B) und (C) werden vorgemischt, abgefüllt und sterilisiert.

Die Herstellung und Sterilisation der Teillösungen oder Gemische von (A), (B) und (C) erfolgt nach den für die Herstellung und Sterilisation für Infusions- und Dialysierlösungen bekannten Verfahren.

Erfindungswesentlich ist es dabei, daß der $pCO_2$ der gemischten Lösungen (A) und (B) uber $87.8 \times 10^2$ Pa (66 mm Hg), [$HCO_3^-$] über 30 mmol/l und folglich der pH-Wert im physiologischen Bereich von 7,2 bis 7,6 liegen.

Einsetzbare organische Puffersysteme sind zum Beispiel: 50 % 0,1 mmol/l Dinatriumcitrat + 50 % 0,1 mmol/l NaOH (pH-Wert ≈ 6,4 ; pK3 ≈ 6,4), oder anstatt Dinatriumcitrat z.B. Malat (pK2 ≈ 5,1) oder Succinat (pK2 ≈ 5,6).

Die gebrauchsfertige erfindungsgemäße Infusions- und Dialysierlösung kann folgende Zusammensetzung aufweisen:

| | |
|---|---|
| $Na^+$ | 120 - 154 mmol/l |
| $K^+$ | 0 - 5 mmol/l |
| $Mg^{++}$ | 0 - 3 mmol/l |
| $Ca^{++}$ | 0,5 - 2,5 mmol/l |
| $Cl^-$ | 50 - 120 mmol/l |
| $HCO_3^-$ | > 30 mmol/l |
| $pCO_2$ | > 66 mm Hg (>$87.8 \times 10^2$Pa) |
| organishes Puffergemish | 10 - 200 mmol/l |
| pH Wert | 7,2 - 7,6 |

Für den Fall, daß die erfindungsgemäße Infusions- und Dialysierlösung osmotische Eigenschaften aufweisen soll, wie dies z.B. beim Einsatz als Peritonealdialyselösung nötig ist, weist sie einen Gehalt einer osmotisch aktiven Substanz oder Gemische solcher osmotisch aktiven Substanzen in entsprechenden Mengen auf. Osmotisch aktive Substanzen sind z.B. Glucose, Fruktose, Galaktose oder andere Zucker, Carbonsäuren, Zuckeralkohole, Glycerin, Zuckerpolymere, Gelatine, Kohlenhydratpolymere, Hydroxyethylstärke, Dextrane, Aminosäuren und deren α-Ketoanaloge und/oder Peptide. In der Praxis wird derzeit insbesondere Glucose in einer Konzentration von 12 bis 50 g/l eingesetzt. Dies führt zu einer Gesamtosmolarität in der Infusions- und Dialysierlösung von etwa 330 bis 700 mosm/l.

Nachfolgendes Beispiel erläutert die Erfindung.

Beispiel:

Es wird eine wässrige 0,1 mol/l Dinatriumcitratlösung in der Weise hergestellt, daß in 1 l Lösung 21,01 g Citronensäuremonohydrat und 200 ml 1 mol/l NaOH enthalten sind. Anschließend wird eine wässrige Lösung Lösung 1) eines Puffergemisches, bestehen aus 0,1 mol/l Dinatriumcitratlösung (a) und 0,1 mol/l NaOH-Lösung (b) im Verhältnis 1 : 1 hergestellt. In diesem Puffergemisch werden 76 mmol/l Natriumhydrogencarbonat gelöst. Der pH-Wert dieses Puffergemisches liegt bei 7,3 bis 7,4. Oder es wird eine weiter wässrige Lösung mit der entsprechenden doppelten Menge Natriumhydrogencarbonat hergestellt (Lösung 1b). Der $pCO_2$ der gemischten Lösung liegt über $87.8 \times 10^2$ Pa (66 mm Hg) , kann aber auch gewünschtenfalls über eine Veränderung des pH-Wertes, gegebenenfalls durch Hinzufügen oder Weglassen von (a) oder (b) auf 7,2 bis 7,6, exakt eingestellt bzw. erhöht werden.

Es wird eine zweite Calciumionen, Glukose und Elektrolyte enthaltende wässrige Lösung hergestellt (Lösung 2).

$$\text{Zusammensetzung:} \quad \begin{array}{ll} CaCl_2 \times 2\ H_2O & 3,5\ \text{mmol/l} \\ MgCl_2 \times 6\ H_2O & 1,0\ \text{mmol/l} \\ NaCl & 100\ \text{mmol/l} \\ Glucose & 30\ \text{g/l} \end{array}$$

Gegebenenfalls kann der pH-Wert dieser Lösung 2 durch hinzufügen einer geringen Menge HCl oder einer anderen Säure auf 5,0 bis 6,8 eingestellt werden, um bei einer eventuellen Hitzesterilisation einer chemischen Umsetzung von Glucose vorzubeugen.

Lösung 1, Lösung 1b (oder ein Gemisch aus Lösung 1 und 1b) und Lösung 2 können

a) pyrogenfrei filtriert und getrennt in geschlossene Behältnisse, vorteilhafterweise in einen Doppelkammerbeutel, dessen Kammern durch eine aufbrechbare Verbindungseinrichtung miteinander in Verbindung gebracht werden können, abgefüllt und danach hitzesterilisiert werden. Es können jedoch auch die Lösungen 1, 1b und 2 separat in einen Dreikammerbeutel abgefüllt werden. Vor Anwendung werden die Lösungen im Doppelkammerbeutel oder Dreikammerbeutel dadurch gemischt, daß nach Aufbrechen der Verbindungseinrichtung durch Druck auf eine Beutelkammerhälfte beide Lösungen im Fall des Doppelkammerbeutelsystems ineinander übergeführt werden. Im Fall des Dreikammerbeutelsystems wird zuerst Lösung 1 und 1b gemischt und anschließend Lösung 2.

Oder

b) in einem geschlossenen Behältnis gemischt, pyrogenfrei filtriert und sterilisiert werden.

Nach a) oder b) ist die Infusions- und Dialysierlösung z.B. als Peritonealdialysierflüssigkeit einsetzbar und weist folgende Zusammensetzung auf:

| | |
|---|---|
| $Na^+$ | 138 mmol/l |
| $Mg^{++}$ | 0,5 mmol/l |
| $Ca^{++}$ | 1,75 mmol/l |
| $Cl^-$ | ca. 70 - 90 mmol/l |
| Glukose | 15 g/l |
| $HCO_3^-$ | > 30 mmol/l |
| $pCO_2$ | > 66 mm Hg (>87.8 x $10^2$Pa) |
| organishes Puffergemisch | 50 mmol/l |
| pH-Wert | 7,3 - 7,4 |
| theoretische Osmolarität | ca. 360 mosm/l |

Die gebrauchsfertige Lösung weist langfristig keine Ausfällungen von $CaCO_3$ auf.

## Patentansprüche

1. Infusions- und Dialysierlösung, enthaltend Bicarbonat und Calciumionen und gegebenenfalls weitere Elektrolyte und Zusatzstoffe, wobei der pH-Wert in einem vorgegebenen Bereich um 7 gehalten wird, **dadurch gekennzeichnet,** daß die gebrauchsfertige Infusions- und Dialysierlösung einen Gehalt eines physiologischen Puffergemisches aus einem organischen Puffersystem und gegebenenfalls einer anorganischen Base oder Säure in einer Konzentration von 10 bis 200 mmol/l aufweist, der den $CO_2$-Partialdruck in dieser Infusions- und Dialysierlösung konstant über 87.8 x $10^2$ Pa (66 mm Hg) hält, und daß der Bicarbonationengehalt wenigstens 30 mmol/l beträgt.

2. Infusions- und Dialysierlösung, enthaltend Bicarbonat- und Calciumionen und gegebenenfalls weitere Elektrolyte und Zusatzstoffe, wobei der pH-Wert in einem vorgegebenen Bereich um 7 gehalten wird, **dadurch gekennzeichnet,** daß die gebrauchsfertige Infusions- und Dialysierlösung einen Gehalt eines physiologischen Puffergemisches aus einem organischen Puffersystem und gegebenenfalls einer anorganischen Base oder Säure in einer Konzentration von 10 bis 200 mmol/l aufweist, der den pH-Wert dieser Infusions- und Dialysierlösung konstant im Bereich von 6,8 bis 7,6 hält, und daß der Bicarbonationengehalt wenigstens 30 mmol/l beträgt.

3. Infusions- und Dialysierlösung nach Anspruch 1 oder 2, **dadurch gekennzeichnet,** daß als physiologisches Puffergemisch organische Carbonsäuren bzw. deren Natrium-, Kalium, Magnesium- oder Calciumsalze, insbesondere Carbonsäuren mit einer Kettenlänge von 3 bis 8 Kohlenstoffatomen, deren pK-Wert im Bereich von 5,0 bis 7,6 liegt und dem korrespondierenden Säuren- bzw. Basenpaar dieser Carbonsäuren oder einer anorganischen Base, ausgewählt aus der Gruppe Hydroxid und Phosphat, insbesondere Natrium-, Kalium-, Magnesium- oder Calciumhydroxid, eingesetzt werden.

4. Infusions- und Dialysierlösung nach einem der Ansprüche 1 bis 3, **dadurch gekennzeichnet,** daß die im physiologischen Puffergemisch zur Anwendung kommenden Carbonsäuren bzw. deren Salze ausgewählt sind aus der Gruppe Citrat, Isocitrat, Oxalsuccinat, $\alpha$-Ketoglutarat, Succinat, Fumarat, Malat, Oxalacetat und Pyruvat oder aus der Gruppe Aminosäuren oder aus der Gruppe der $\alpha$-Ketoanalogen von Aminosäuren.

5. Infusions- und Dialysierlösung nach einem der Ansprüche 1 bis 4,
   **dadurch gekennzeichnet,**
   daß der pH-Wert im physiologischen Bereich von 7,2 bis 7,6, vorzugsweise 7,3 bis 7,4 liegt.

6. Verfahren zur Herstellung einer Infusions- und Dialysierlösung nach einem der Ansprüche 1 bis 5,
   **dadurch gekennzeichnet,**
   daß in eine, ein organisches Puffersystem und gegebenenfalls eine anorganische Base oder Säure als physiologisches Puffergemisch enthaltende wässrige Lösung eine Bicarbonationen enthaltende Lösung und anschließend eine Calciumionen enthaltende Lösung gegeben werden.

7. Verfahren zur Herstellung einer Infusions- und Dialysierlösung nach einem der Ansprüche 1 bis 5,
   **dadurch gekennzeichnet,**
   daß in einer, ein organisches Puffersystem und gegebenenfalls eine an organiche Base oder Säure als physiologisches Puffergemisch enthaltende wässrige Lösung Bicarbonat- und Calciumionen direkt in Form ihrer Salze gelöst werden.

8. Verfahren zur Herstellung einer Infusions- und Dialysierlösung nach einem der Ansprüche 1 bis 5,
   **dadurch gekennzeichnet,**
   daß ein ein organisches Puffersystem und gegebenenfalls eine an organische Base oder Säure enthaltendes physiologisches Puffergemisch als wässrige Lösung oder als Salze in eine Bicarbonationen enthaltende Lösung gegeben wird.

## Claims

1. Infusion and dialysing solution containing bicarbonate and calcium ions, and optionally additional electrolytes and additives by means of which the pH is maintained in a predetermined range around 7, characterised in that the ready to use infusion and dialysing solution contains a physiological buffer mixture of an organic buffer system and optionally an inorganic base or acid at a concentration of 10 to 200 mmol/l, which maintains the partial pressure of $CO_2$ in this infusion and dialysing solution constantly over 87.8 x $10^2$ Pa (66 mm Hg), and that the bicarbonate ion content is at least 30 mmol/l.

2. Infusion and dialysing solution containing bicarbonate and calcium ions, and optionally other additives by means of which the pH is maintained in a predetermined range around 7, characterised in that the ready to use infusion and dialysing solution contains a physiological buffer mixture of an organic buffer system and optionally an inorganic base or acid at a concentration of 10 to 200 mmol/l, which maintains the pH of this infusion and dialysing solution constantly in the range of 6.8 to 7.6, and that the bicarbonate ion content is at least 30 mmol/l.

3. Infusion and dialysing solution according to Claims 1 or 2, characterised in that the materials used as the physiological buffer mixture are organic carboxylic acids or their sodium, potassium, magnesium or calcium salts, especially carboxylic acids with a chain length of 3 to 8 carbon atoms, the pK value of which is in the range 5.0 to 7.6, and the corresponding acid or base pair of these carboxylic acids or an inorganic base, selected from the group of hydroxide and phosphate, especially sodium, potassium, magnesium or calcium hydroxide.

4. Infusion and dialysing solution according to one of Claims 1 to 3, characterised in that the carboxylic acids or their salts used in the physiological buffer mixture are selected from the group comprising citrate, isocitrate, oxalsuccinate, $\alpha$-ketoglutarate, succinate, fumarate, malate, oxalacetate and pyruvate or from the aminoacids group or from the group of the $\alpha$-ketoanalogues of aminoacids.

5. Infusion and dialysing solution according to one of the Claims 1 to 4, characterised in that the pH is in the physiological range of 7.2 to 7.6, preferably 7.3 to 7.4.

6. Process for the manufacture of an infusion and dialysing solution according to one of the Claims 1 to 5, characterised in that a solution containing bicarbonate ions and a solution containing calcium ions are added sequentially to an aqueous solution containing an organic buffer system and, optionally, an inorganic base or acid as physiological buffer mixture.

7. Process for the manufacture of an infusion and dialysing solution according to one of the Claims 1 to 5, characterised in that bicarbonate and calcium ions are dissolved directly as their salts in an aqueous solution containing an organic buffer system and, optionally, an inorganic acid or base as physiological buffer mixture.

8. Process for the manufacture of an infusion and dialysing solution according to one of the Claims 1 to 5, characterised in that a physiological buffer mixture containing an organic buffer system and optionally an inorganic base or acid is added, as an aqueous solution or as salts. to a solution containing bicarbonate ions.

**Revendications**

1. Solution de perfusion et de dialyse contenant des ions de bicarbonate et de calcium et éventuellement d'autres électrolytes et additifs, le pH étant maintenu dans une plage prédéterminée aux environs de 7, caractérisée en ce que la solution de perfusion et de dialyse prête à l'emploi contient un mélange tampon physiologique constitué d'un système tampon organique et éventuellement d'une base ou d'un acide minéral selon une concentration de 10 à 200 mmoles/l, qui maintient constamment la pression partielle de $CO_2$ dans cette solution de perfusion et de dialyse au-dessus de $87,8 \times 10^2$ Pa (66 mm Hg), et en ce que la teneur en ions de bicarbonate est d'au moins 30 mmoles/l.

2. Solution de perfusion et de dialyse contenant des ions de bicarbonate et de calcium et éventuellement d'autres électrolytes et additifs, le pH étant maintenu dans une plage prédéterminée aux environs de 7, caractérisée en ce que la solution de perfusion et de dialyse prête à l'emploi contient un mélange tampon physiologique constitué d'un système tampon organique et éventuellement d'une base ou d'un acide minéral selon une concentration de 10 à 200 mmoles/l, qui maintient constamment le pH dans cette solution de perfusion et de dialyse dans la plage de 6,8 à 7,6, et en ce que la teneur en ions de bicarbonate est d'au moins 30 mmoles/l.

3. Solution de perfusion et de dialyse selon la revendication 1 ou 2, caractérisée en ce que l'on utilise à titre de mélange tampon physiologique des acides carboxyliques organiques ou leurs sels de sodium, de potassium, de magnésium ou de calcium, en particulier des acides carboxyliques ayant une longueur de chaîne de 3 à 8 atomes de carbone, dont le pK se situe dans la plage de 5,0 à 7,6, et la paire correspondante d'acides ou de bases de ces acides carboxyliques ou une base minérale choisie entre les hydroxydes et les phosphates, en particulier l'hydroxyde de sodium, de potassium, de magnésium ou de calcium.

4. Solution de perfusion et de dialyse selon l'une quelconque des revendications 1 à 3, caractérisée en ce que les acides carboxyliques susceptibles d'être utilisés dans le mélange physiologique, ou leurs sels, sont choisis parmi les citrates, les isocitrates, les oxalsuccinates, les alphacétoglutarates, les succinates, les fumarates, les maléates, les oxalacétates et les pyruvates ou parmi les amino-acides ou les alaphacéto-analogues des amino-acides.

5. Solution de perfusion et de dialyse selon l'une quelconque des revendications 1 à 4, caractérisée en ce que le pH se situe dans la plage physiologique de 7,2 à 7,6, de préférence de 7,3 à 7,4.

6. Procédé de préparation d'une solution de perfusion et de dialyse selon l'une quelconque des revendications 1 à 5, caractérisé en ce que l'on ajoute une solution contenant des ions de bicarbonate et ensuite une solution contenant des ions de calcium à une solution aqueuse contenant un système tampon organique et éventuellement une base ou un acide minéral à titre de mélange tampon physiologique.

7. Procédé de préparation d'une solution de perfusion et de dialyse selon l'une quelconque des revendications 1 à 5, caractérisé en ce que l'on dissout dans une solution aqueuse contenant un système tampon organique et éventuellement une base ou un acide minéral à titre de mélange tampon physiologique des ions de bicarbonate et de calcium directement sous la forme de leurs sels.

8. Procédé de préparation d'une solution de perfusion et de dialyse selon l'une quelconque des revendications 1 à 5, caractérisé en ce que l'on ajoute un mélange tampon physiologique contenant un système tampon organique et éventuellement une base ou un acide minéral sous la forme d'une solution aqueuse ou des sels à une solution contenant des ions de bicarbonate.